Europäisches Patentamt

European Patent Office

Office européen des brevets

(19)

(11) Numéro de publication : **0 446 333 B1**

(12) **FASCICULE DE BREVET EUROPEEN**

(45) Date de publication du fascicule du brevet :
28.07.93 Bulletin 93/30

(51) Int. Cl.⁵ : **G01V 1/00**, G01N 23/223

(21) Numéro de dépôt : **90914740.7**

(22) Date de dépôt : **02.10.90**

(86) Numéro de dépôt international :
**PCT/FR90/00701**

(87) Numéro de publication internationale :
**WO 91/05272 18.04.91 Gazette 91/09**

(54) **SYSTEME DE DETECTION DE SUBSTANCES ET EN PARTICULIER D'EXPLOSIFS, PAR IRRADIATION NEUTRONIQUE DE CEUX-CI.**

(30) Priorité : **03.10.89 FR 8912917**

(43) Date de publication de la demande :
**18.09.91 Bulletin 91/38**

(45) Mention de la délivrance du brevet :
**28.07.93 Bulletin 93/30**

(84) Etats contractants désignés :
**BE DE GB IT NL**

(56) Documents cités :
**EP-A- 0 227 497**
**FR-A- 2 201 765**
**US-A- 3 997 787**
**US-A- 4 171 485**

(73) Titulaire : **COMMISSARIAT A L'ENERGIE ATOMIQUE**
**31-33, rue de la Fédération**
**F-75015 Paris (FR)**

(72) Inventeur : **GRENIER, Gérard**
**33, avenue Charles-Emmanuel**
**F-94450 Limeil-Brevannes (FR)**
Inventeur : **RAMBAUT, Michel**
**57 H, rue de la Hacquinière**
**F-91440 Bures-sur-Yvette (FR)**

(74) Mandataire : **Mongrédien, André et al**
**c/o BREVATOME 25, rue de Ponthieu**
**F-75008 Paris (FR)**

EP 0 446 333 B1

Jouve, 18, rue Saint-Denis, 75001 PARIS

## Description

La présente invention concerne un système de détection de substances et en particulier d'explosifs, par irradiation neutronique de ceux-ci. Elle s'applique notamment au contrôle de bagages, en particulier dans les aéroports.

On connaît déjà des systèmes de détection d'explosifs, utilisant une irradiation de ceux-ci par des neutrons. On peut consulter notamment à ce sujet les documents FR-A-2201765 et EP-A-0227497.

On rappelle à ce propos les notions suivantes :

Il existe trois processus de production de photons gamma par interaction de neutrons avec des noyaux :

- la capture d'un neutron par un noyau, engendrant un isotope qui se désexcite par émission d'un rayonnement gamma, appelé rayonnement gamma de capture ("capture gamma radiation" dans les publications en langue anglaise), cette interaction ayant essentiellement lieu avec des neutrons thermiques ("thermal neutrons" dans les publications en langue anglaise),
- la diffusion inélastique d'un neutron sur un noyau qui se désexcite par émission d'un rayonnement gamma appelé rayonnement gamma prompt ("prompt gamma radiation" dans les publications en langue anglaise), cette interaction existant seulement avec des neutrons rapides ("fast neutrons" dans les publications en langue anglaise) ayant assez d'énergie (c'est-à-dire une énergie au moins égale à celle du rayonnement gamma prompt), et
- l'activation d'un noyau par un neutron thermique ou rapide, qui crée un noyau radioactif ayant une certaine durée de vie et se désintégrant en émettant un rayonnement gamma appelé rayonnement gamma d'activation ("activation gamma radiation" dans les publications en langue anglaise).

Par ailleurs, les explosifs usuels comportent des atomes de carbone, d'hydrogène, d'oxygène et d'azote et les éléments les plus intéressants pour détecter la présence d'un explosif sont l'azote et l'oxygène, notamment à cause de la séparabilité des raies (ou pics) gammas, créés par diffusion inélastique de neutrons rapides, l'énergie des photons gamma se répartissant entre 2,3129 MeV et 6,1304 MeV.

L'utilisation du rayonnement gamma de capture de l'azote à 10,83 MeV est également intéressante du fait du bon rapport signal/bruit dans l'intervalle d'énergie proche de 10 MeV.

Les systèmes de détection connus, mentionnés plus haut, présentent l'inconvénient de donner lieu à une probabilité trop élevée de défaut de détection (de l'ordre de $10^{-2}$ ou plus) lors de contrôles à cadences élevées et pour de faibles quantités d'explosif.

La présente invention a pour but de remédier à cet inconvénient en proposant un système de détection de substances et en particulier d'explosifs, qui permet d'abaisser cette probabilité de défaut de détection et même d'atteindre des valeurs très faibles pour celle-ci ($10^{-4}$, $10^{-10}$ ou même moins de $10^{-10}$) en utilisant des moyens de détection de haute résolution pour détecter le rayonnement gamma.

De façon précise, la présente invention a pour objet un système de détection d'une substance susceptible d'être contenue dans un objet, système caractérisé en ce qu'il comprend :

- des moyens d'irradiation de l'objet par des neutrons (thermiques et/ou rapides),
- des moyens de détection du rayonnement gamma susceptible d'être alors émis par l'objet, et
- des moyens électroniques de traitement des signaux fournis par les moyens de détection, ces moyens électroniques de traitement étant prévus pour
  . compter les photons gamma correspondant à chaque raie d'un ensemble de raies caractéristiques d'au moins un élément chimique de la substance,
  . déterminer, pour chaque raie i, une probabilité de fausse détection de l'élément chimique associé à cette raie, c'est-à-dire la probabilité $PF_i$ pour que le signal détecté, correspondant à cette raie i, soit dû à un bruit de fond,
  . déterminer le produit de ces probabilités de fausse détection,
  . comparer ce produit à un seuil fixé par les utilisateurs du système, et
  . avertir ces utilisateurs si le produit est inférieur au seuil fixé par eux, l'objet étant alors présumé contenir la substance.

Des éléments importants de l'invention, permettant de diminuer la probabilité de défaut de détection par rapport aux systèmes connus, sont donc :

- le choix d'un ou d'une pluralité d'éléments chimiques que contient la substance que l'on veut détecter, par exemple l'azote seul ou l'azote et l'oxygène ou encore l'azote, l'oxygène et le carbone dans le cas d'un explosif,
- le choix d'une pluralité de raies qui sont caractéristiques du ou des éléments choisis et dont on compte les photons gamma correspondants, par exemple, dans le cas d'un explosif, les raies à 5,106 MeV et à 10,83 MeV de l'azote ou bien l'une et/ou l'autre des raies précédentes ainsi qu'une ou plusieurs raies caractéristiques de l'oxygène et, éventuellement, en plus, des raies respectivement caractéristiques

2

d'éléments tels que le carbone, l'hydrogène, le chlore (explosif chloré), et

- l'évaluation de la probabilité de fausse détection relative à chacune de ces raies (ou d'une quantité d'information liée à cette probabilité).

Chaque probabilité de fausse détection PFi peut être déterminée par la formule :

$$PFi = e^{-M'2i} \sum_{j=Ni+1}^{+\infty} \frac{M'2i^j}{j!}$$

où Ni représente le comptage, pendant un temps Dt, qui correspond à la raie i et qui est dû à des réactions nucléaires induites par des neutrons et aussi à un bruit de fond M'2i relatif à la raie i, pendant le temps Dt.

Chaque bruit de fond M'2i peut être déterminé en faisant la somme d'un bruit de fond physique M2i, relatif à la raie i, et de la borne supérieure Ci de comptages relatifs à cette raie i, pendant le temps Dt, sur des objets qui sont susceptibles de contenir la substance mais dont on est sûr qu'ils n'en contiennent pas.

Selon un premier mode de réalisation particulier du système objet de l'invention, les moyens électroniques de traitement sont prévus pour :

. déterminer, pour chaque raie i, une quantité d'information Ii relative à cette raie i et définie par la formule :

$$Ii = -\log \frac{PFi}{1 - PFi}$$

(après avoir déterminé Ni puis PFi),

. déterminer la somme de ces quantités d'information,

. comparer cette somme à un seuil fixé par les utilisateurs du système, et

. avertir ces utilisateurs si cette somme est supérieure au seuil auquel elle est comparée, l'objet étant alors présumé contenir la substance.

Selon un deuxième mode de réalisation particulier, permettant un temps de calcul plus court et donc un temps de contrôle plus court de l'objet, les moyens électroniques de traitement sont prévus pour :

. déterminer, pour chaque raie i, une quantité d'information Ii relative à cette raie i et définie par la formule :

$$Ii = K(M'2i) . \frac{N1i^{a(M'2i)}}{M'2i^{1/2}}$$

(après avoir déterminé Ni mais sans avoir à déterminer PFi), formule où K et a sont des fonctions mémorisées d'un bruit de fond M'2i relatif à la raie i et N1i est la partie entière de la différence Ni-M'2i, Ni représentant le comptage, pendant un temps Dt, qui correspond à la raie i et qui est dû à des réactions nucléaires induites par des neutrons et aussi au bruit de fond M'2i relatif à la raie i, pendant le temps Dt,

. déterminer la somme de ces quantités d'information,

. comparer cette somme à un seuil fixé par les utilisateurs du système, et

. avertir ces utilisateurs si cette somme est supérieure au seuil auquel elle est comparée, l'objet étant alors présumé contenir la substance.

Le système objet de l'invention s'applique aussi bien à une configuration "fermée" qu'à une configuration "ouverte" :

Les moyens d'irradiation peuvent comprendre une source de neutrons rapides et une enceinte de thermalisation de ces neutrons rapides, dans laquelle se trouve la source et qui est prévue pour recevoir l'objet, ou peuvent comprendre une source de neutrons rapides qui est prévue pour irradier l'objet (directement, sans enceinte de thermalisation).

Afin de pouvoir contrôler l'ensemble de l'objet, les moyens de détection peuvent comprendre une pluralité de détecteurs de rayonnement gamma, les moyens électroniques de traitement comprenant une pluralité de chaînes de détection, respectivement associées aux détecteurs, la configuration de ces détecteurs pouvant être matricielle.

Selon un mode de réalisation préféré du système objet de l'invention, permettant de diminuer fortement la probabilité de défaut de détection par rapport aux systèmes connus, les moyens de détection du rayonnement gamma sont des moyens de détection de haute résolution.

A cet effet, on peut par exemple utiliser un ou une pluralité de détecteurs en germanium de haute pureté.

De préférence, les moyens d'irradiation comprenant une source de neutrons, les moyens de détection sont protégés du rayonnement direct de la source et collimatés vers l'objet.

Dans une réalisation préférée de l'invention, les moyens d'irradiation comprennent une source pulsée de

3

neutrons, prévue pour fournir des bouffées de neutrons, et les moyens électroniques de traitement coopèrent avec les moyens de détection pour faire des mesures dans des intervalles de temps où l'on est sûr de détecter seulement l'une des catégories de photons gamma engendrés lors de l'irradiation de l'objet par des neutrons.

Avec cette source de neutrons pulsée, on réalise par exemple une détection synchrone des photons gamma prompts et une détection des photons gamma de capture entre les bouffées de neutrons.

Ceci permet d'obtenir, pour chaque type de détection, un rapport signal/bruit nettement supérieur à celui que l'on obtient avec une source continue de neutrons.

Dans la présente invention, les moyens d'irradiation comprennent, de préférence, une source de neutrons de 14 MeV engendrés par des réactions de fusion.

En effet, une source de neutrons d'une telle énergie permet de contrôler la plupart des éléments chimiques.

Enfin, dans un mode de réalisation particulier de l'invention, approprié à la détection d'explosif, les moyens électroniques de traitement sont prévus pour compter les photons gamma d'un ensemble de raies qui sont respectivement caractéristiques de l'azote, de l'oxygène et éventuellement du carbone, dans le cas d'explosifs nitrés, ou d'autres éléments dans le cas d'autres explosifs.

La présente invention sera mieux comprise à la lecture de la description suivante, d'exemples de réalisation donnés à titre purement indicatif et nullement limitatif, en référence aux dessins annexés sur lesquels :

- la figure 1 est un graphique illustrant les variations de la quantité d'information en fonction du taux moyen de signal, à taux de bruit physique constant et pour divers taux de bruit physique,
- la figure 2 est un graphique permettant de comparer l'une à l'autre deux expressions de la quantité d'information,
- la figure 3 est un graphique montrant la décroissance monotone, en fonction du taux de bruit physique, de chacune de ces deux expressions de la quantité d'information,
- la figure 4 est une vue en perspective schématique d'un mode de réalisation particulier du système objet de l'invention, utilisant une pluralité de détecteurs fixes de rayonnement gamma et une enceinte de thermalisation,
- la figure 5 illustre schématiquement des moyens électroniques de traitement qui sont utilisables pour traiter les impulsions fournies par ces détecteurs,
- la figure 6 illustre schématiquement d'autres moyens électroniques de traitement qui sont également utilisables pour traiter les impulsions fournies par les détecteurs représentés sur la figure 4,
- la figure 7 est une vue schématique et partielle d'un autre mode de réalisation particulier du système objet de l'invention, utilisant un détecteur mobile de rayonnement gamma, et
- la figure 8 est une vue schématique d'un autre mode de réalisation particulier du système objet de l'invention, de configuration "ouverte".

Dans ce qui suit, on suppose que l'on veut contrôler des bagages qui sont susceptibles de contenir des explosifs, par exemple des explosifs nitrés.

On ne peut disposer par bagage que d'une durée de temps limitée afin de pouvoir assurer un débit suffisant, mesuré en nombre de bagages par heure. Cette durée de temps est par exemple de l'ordre de quelques secondes.

Un bagage est donc amené à un système conforme à l'invention où il est irradié par des neutrons et l'on effectue un comptage, pendant une durée Dt, de photons gamma parmi lesquels figurent, bien entendu, des photons gamma émis par l'objet à la suite de l'irradiation de ce dernier.

A cette durée Dt correspond un nombre moyen d'événements M2 qui est dû à diverses causes physiques (mouvement propre du ou des détecteurs de rayonnement gamma, rayonnement du milieu environnant, rayonnement cosmique ...) et que l'on appelle "bruit de fond physique".

Pour cette même durée Dt, il existe un comptage moyen M1, que l'on peut attribuer à la présence d'un produit suspect, de la matière azotée par exemple.

Des essais effectués sur un grand nombre de bagages montrent qu'au bagage typique du voyageur "moyen" correspond un comptage maximum Ci sous un pic i, comptage maximum qui est attribuable par exemple à de la matière azotée (le pic i étant, par exemple, le pic à 10,83 MeV de l'azote) sans que ce bagage contienne une quelconque quantité d'explosif.

On attribue alors à la présence d'explosif tout comptage qui est supérieur à la somme M2i+Ci, M2i étant la valeur du comptage due au bruit de fond physique sous le pic i pendant le temps Dt. On considère alors, pour la détection d'une quantité anormale d'azote attribuable à un explosif, qu'il existe un bruit de fond M'2i tel que :

$$M'2i = M2i + Ci \quad (1)$$

Conformément à la présente invention, on choisit à l'avance d'examiner un certain nombre de raies i, i allant de 1 à n, dans le spectre de rayonnement gamma et l'on caractérise la présence de chaque raie i par la probabilité PFi pour que le signal détecté soit dû au bruit de fond ou à une quantité de matière suspecte inférieure à la borne supérieure de la quantité normale, en d'autres termes, pour qu'il y ait fausse détection.

On fait correspondre au comptage Ni "brut", sous le pic i, et au comptage moyen de bruit M'2i, une probabilité de fausse détection PFi telle que :

$$PFi = e^{-M'2i} \sum_{j=Ni+1}^{+\infty} \frac{M'2i^j}{j!} \quad (2)$$

De plus, on considère la valeur N1i égale à la partie entière de la différence entre le comptage brut Ni sous le pic i et M'2i, c'est-à-dire :

$$N1i = E(Ni - M'2i) \quad (3)$$

en notant $E(x)$ la partie entière du nombre x.

On fait correspondre à la valeur N1i et à la valeur M'2i du bruit de fond, une probabilité de défaut de détection PDi qui caractérisé le défaut inévitable du système de ne pas détecter ce qu'il devrait détecter, cette probabilité de défaut de détection étant telle que :

$$PDi = e^{-(N1i+M'2i)} \sum_{k=0}^{E(M'2i)} \frac{(N1i+M'2i)^k}{k!} \quad (4)$$

On peut montrer que, à bruit de fond M'2i croissant, PDi et PFi tendent vers une même valeur, PDi restant supérieur à PFi, la probabilité PDi constituant un élément d'appréciation fondamental pour connaître l'aptitude du système à ne pas manquer un bagage qui comporterait une charge d'explosif.

Cependant, dans la présente invention, on utilise la probabilité PFi étant donné son importance "opérationnelle" (et le fait que PDi et PFi tendent vers ladite même valeur).

Dans la présente invention, on peut également mesurer l'information obtenue pendant le temps Dt au moyen d'une fonction appelée "quantité d'information" et notée Ii pour la raie i, cette quantité Ii dépendant de la probabilité de fausse détection PFi obtenue au cours d'un stockage de photons gamma.

Chaque quantité d'information satisfait aux conditions suivantes :
- additivité des quantités d'information obtenues lors de deux stockages séparés, effectués dans les mêmes conditions, et
- nullité de la quantité d'information avant tout stockage.

Les probabilités de fausse détection PF' et PF" sur un phénomène particulier, au cours de deux comptages distincts, sont indépendantes, si bien que la probabilité de fausse détection PF résultant des deux comptages considérés globalement, a pour expression :

$$PF = PF' \times PF'' \quad (5)$$

On choisit alors l'expression suivante pour la quantité d'informations Ii :

$$Ii = -\log \frac{PFi}{1 - PFi} \quad (6)$$

Avant la mesure, les probabilités de vraie et de fausse détection sont a priori égales à 0,5 et l'expression (6) de la quantité d'information est alors nulle. Elle est également nulle dans le cas où le comptage Ni est égal au comptage moyen de bruit de fond M2i car PFi est alors égal à 0,5. La quantité d'information Ii est positive si le comptage Ni est supérieur à M2i et négative si Ni est inférieur à M2i.

Il est à noter qu'on pourrait changer le signe de la quantité d'information en changeant de critère (obtention d'une quantité d'information Ii' sur la non-existence d'un phénomène particulier, avec Ii'=Ii).

Au sujet de cette notion de quantité d'information, on se reportera aux documents suivants :
- Article de M.L. RAMBAUT, intitulé "Détection d'un rayonnement corpusculaire et information" et publié dans Nuclear Instruments and Methods in Physics Research A248 (1986) p.471-482 North Holland, Amsterdam.
- EP-A-0200596
- EP-A-0202980
- demande de brevet français n°8712970 du 18 septembre 1987.

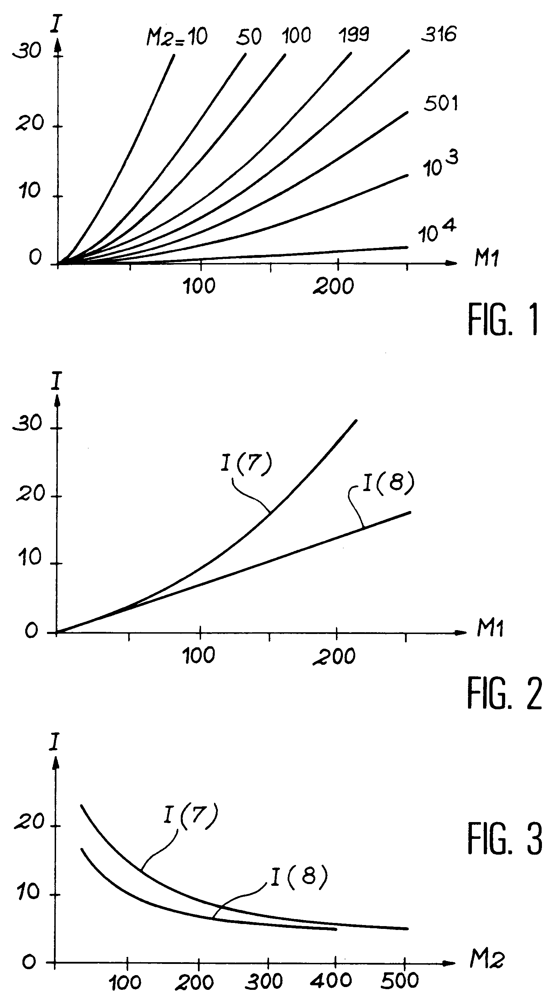

Sur la figure 1, on a tracé la courbe des variations de la quantité d'information I en fonction du taux moyen de signal M1 exprimé en nombre d'événements pendant le temps Dt, et à bruit, noté M2, constant et exprimé en nombre d'événements pendant le temps Dt, pour plusieurs valeurs de M2 (10, 50, 100, 199, 316, 501, $10^3$ et $10^4$), la base des logarithmes étant égale à 10 et la quantité d'information étant exprimée en "digits".

Le réseau de courbes de la figure 1 montre bien quantitativement l'avantage qu'il y a à placer le ou les détecteurs de rayonnement gamma là où le bruit M2 est minimal.

Le système objet de l'invention peut comprendre une source de neutrons de fission, par exemple une source de Californium 252.

Cependant, ce système comprend avantageusement une source pulsée de neutrons de fusion de 14 MeV ; en effet, si la mesure n'est faite que dans l'intervalle de temps où l'on est sûr de détecter l'une des trois catégories de photons gamma mentionnées plus haut, on peut réduire proportionnellement la durée de stockage et donc la valeur du bruit de fond.

Au lieu d'utiliser la formule (6) pour la quantité d'information Ii, on peut utiliser une formule semi-phénoménologique que l'on peut déduire des formules (2) et (6) et qui est la suivante :

$$Ii = K(M'2i) \cdot \frac{N1i^{a(M'2i)}}{M'2i^{1/2}} \quad (7)$$

où K et a sont des fonctions de M'2i.

Cette formule (7), qui est fondée, par l'intermédiaire de la formule (2), sur le calcul des probabilités, facilite le dialogue entre le système objet de l'invention et les utilisateurs de ce dernier.

D'autre part, elle est préférable à une formule du genre :

$$Ii = \frac{N1i}{M'2i^{1/2}} \quad (8)$$

En effet, en considérant la figure 2, sur laquelle une quantité d'information I(7) selon la formule (7) et une quantité d'information I(8) selon la formule (8), exprimées en "digits", sont représentées en fonction d'un taux de signal M1, pour un bruit M2 fixé, égal à 199 (événements dûs au bruit physique pendant le temps Dt), on voit que l'expression de la formule (7) fournit plus d'information que l'expression de la formule (8).

Dans la présente invention, le choix des moyens de détection du rayonnement gamma est important.

On pourrait choisir un ou des détecteurs de faible résolution, utilisant par exemple des cristaux d'iodure de sodium, ou de très faible résolution, utilisant par exemple des cristaux de germanate de bismuth, de coût relativement faible. Cependant, de tels détecteurs ne fournissent pas une garantie suffisante aux utilisateurs du système, étant donné que leur emploi conduit à une probabilité de défaut de détection d'explosif PD trop élevée.

C'est pourquoi on utilise de préférence, dans la présente invention, un ou des détecteurs de haute résolution, comportant par exemple un cristal de germanium intrinsèque. De tels détecteurs permettent de séparer tous les pics de photons gamma utiles (dont certains ne seraient même pas visibles sur un spectre obtenu avec un détecteur de faible résolution) et de disposer sous un pic gamma, d'un rapport signal/bruit maximum et donc d'obtenir une quantité d'information maximum dans un temps donné Dt.

Comme on le verra par la suite, l'utilisation de tels détecteurs de haute résolution, en association avec l'utilisation des probabilités de fausse détection (ou des quantités d'information du genre de celles de la formule (6) ou de la formule (7)), permet d'abaisser considérablement la probabilité de défaut de détection de l'explosif, jusqu'à des niveaux bien inférieurs à $10^{-2}$.

Certes, on pourrait utiliser des détecteurs de faible résolution tout en augmentant le flux de neutrons par unité de temps. Cependant, ceci conduirait à une augmentation de l'importance des problèmes de radio-protection inhérents aux systèmes de détection d'explosif par irradiation neutronique et conduirait surtout à des empilements dans les circuits électroniques analogiques de traitement des signaux fournis par les détecteurs de rayonnement gamma.

Pour diminuer PF et PD et pour augmenter la quantité d'information, on augmente donc la résolution des moyens de détection, ce qui entraîne une réduction du bruit dans la même proportion.

Si l'on suppose que la densité de probabilité sous un pic i, en fonction de l'énergie des photons gamma, est gaussienne, l'écart-type s du comptage des événements est proportionnel au bruit de fond physique M2i sous le pic. Donc, en supprimant l'indice i pour plus de simplicité et en considérant deux détecteurs repérés seulement par des indices A et B, on peut écrire :

$$sA/sB = M2A/M2B \quad (9)$$

En conséquence, les efficacités des détecteurs étant supposées identiques et les comptages dûs au signal étant aussi, de ce fait, identiques dans les deux détecteurs, si les quantités d'information IA et IB, correspondant respectivement aux détecteurs A et B, sont exprimées par la formule (8), on obtient que chacune de ces quantités d'information IA et IB, obtenues pendant le même temps Dt, est inversement proportionnelle à la racine carrée de l'écart-type correspondant.

En revanche, si l'on utilise la formule (7) pour calculer les quantités d'information IA et IB, si le taux de signal M1A est égal au taux de signal M1B et si le bruit M2A est inférieur au bruit M2B, on obtient que le rapport IA/IB est inférieur au rapport $sB^{1/2}/sA^{1/2}$. Ceci résulte du fait que la quantité d'information exprimée par la formule (7) décroît de façon monotone en fonction du bruit tout en restant supérieure à la quantité d'information exprimée par la formule (8), comme on le voit sur la figure 3 où l'on a choisi une valeur particulière du comptage dans la voie signal, à savoir M1=100 événements durant le temps Dt.

A titre d'exemple, on considère le cas de la raie à 10,83 MeV de l'azote, on prend un écart-type de 500 keV environ pour le détecteur d'indice B, ce qui caractérise typiquement la faible résolution d'un détecteur en germanate de bismuth ; on considère que la largeur de la bande d'énergie autour de 10,83 MeV, dans laquelle sont détectés les photons gamma, est égale à 1,3 MeV ; pour le détecteur d'indice A, on prend un écart-type de 10 keV et une bande d'énergie de largeur 20 keV autour de 10,83 MeV, ce qui correspond typiquement à un détecteur en germanium intrinsèque ; on obtient alors un rapport IA/IB supérieur à 8 ; plus précisément, si l'on applique la formule (2), à des valeurs de $10^{-2}$ environ pour PF et PD (M2=120 et M1=130 événements pendant le temps Dt), valeurs qui sont accessibles à l'aide d'un détecteur de faible résolution, il correspond, pour un détecteur de haute résolution, une valeur proche de $10^{-26}$ et par conséquent une sécurité de détection quasiment absolue.

On notera également que des moyens de détection de haute résolution permettent d'augmenter le débit des bagages par unité de temps, en réduisant la durée d'emmagasinage, en vue d'obtenir une valeur intermédiaire entre $10^{-2}$ et $10^{-26}$ pour PF et pour PD.

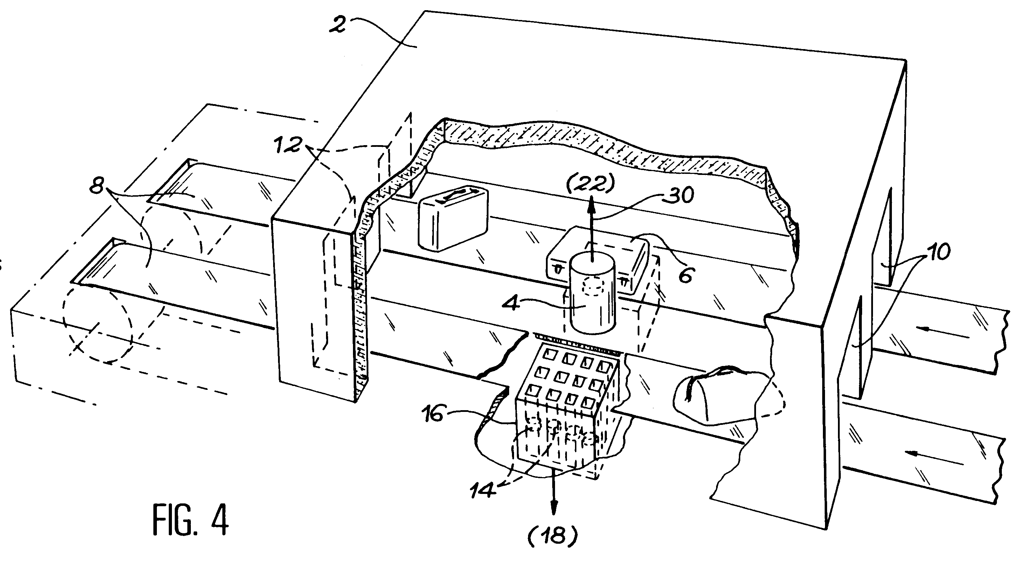

La figure 4 est une vue en perspective schématique et partielle d'un système conforme à l'invention, destiné à contrôler des bagages pour s'assurer qu'il ne contiennent pas d'explosif.

Le système représenté sur la figure 4 comprend une enceinte 2 de thermalisation de neutrons et, dans cette enceinte, au milieu de cette dernière par exemple, une source 4 de neutrons rapides, par exemple une source de neutrons de 14 MeV.

L'enceinte 2 est faite d'une matière apte à thermaliser les neutrons rapides, par exemple une matière hydrogénée telle que le polystyrène.

Pour gagner du temps, le système est prévu pour permettre le contrôle de deux bagages 6 en même temps et comprend à cet effet deux tapis roulants 8 qui traversent l'enceinte 2 et qui permettent de faire circuler les bagages 6 de part et d'autre de la source 4, l'enceinte 2 comportant, pour chaque tapis roulant 8, une entrée 10 permettant l'entrée des bagages dans l'enceinte et une sortie 12 permettant aux bagages de quitter l'enceinte.

Les tapis roulants 8 sont faits d'un matériau mince qui présente une faible absorption vis-à-vis du rayonnement gamma et contient le moins possible d'éléments parmi ceux que l'on veut détecter.

Le système représenté sur la figure 4 comprend également des moyens de détection du rayonnement gamma émis par les bagages lorsqu'ils sont irradiés par des neutrons. Ces moyens de détection comprennent, pour chaque tapis roulant 8, une matrice de détecteurs 14, de préférence à haute résolution.

Chaque matrice de détecteurs 14 est placée sous le tapis roulant 8 correspondant, au niveau de la source 4, de telle façon que chaque bagage 6 à contrôler, qui circule sur ce tapis roulant et y repose de préférence par sa plus grande surface, puisse être mis en présence de la source 4 et de la matrice de détecteurs correspondant à ce tapis roulant.

Dans une variante non représentée, les matrices de détecteurs sont placées, non pas sous leurs tapis roulants respectifs, mais de part et d'autre de la source, entre les deux tapis.

On voit sur la figure 4 que chaque détecteur 14 est protégé du rayonnement direct de la source de neutrons et collimaté vers le bagage 6 à contrôler. A cet effet, on utilise un bloc ou blindage 16 qui est fait d'un matériau apte à arrêter les neutrons et dans lequel sont prévus des logements qui accueillent chacun un détecteur 14.

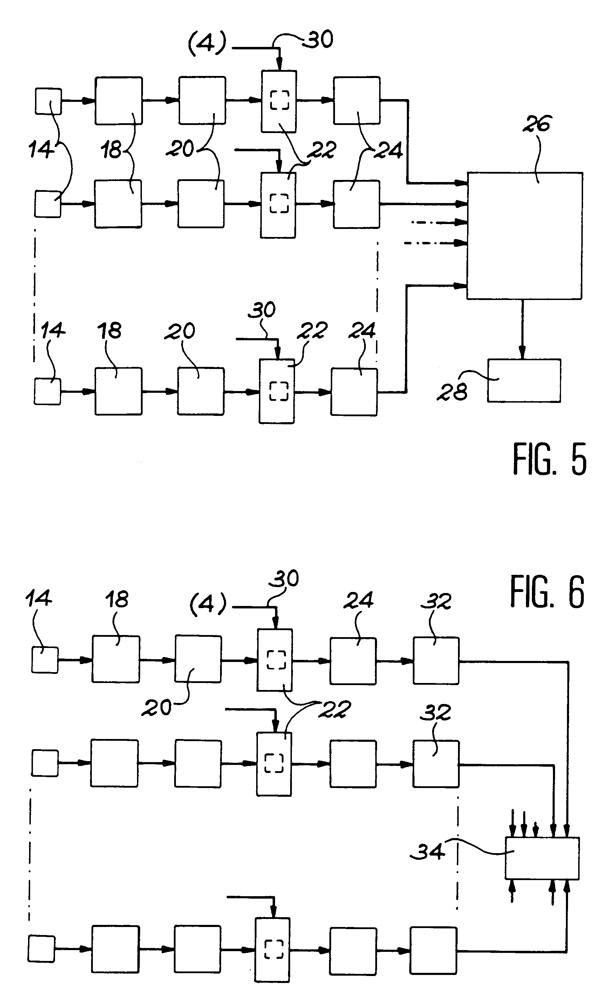

La figure 5 illustre schématiquement des moyens électroniques de traitement des impulsions fournies par les détecteurs 14 (qui fonctionnent simultanément). Ces moyens électroniques de traitement comprennent une pluralité de chaînes de détection. Chaque détecteur 14 est associé à une chaîne de détection qui comprend successivement, à la suite du détecteur 14 associé, un préamplificateur 18, un amplificateur proportionnel 20, un discriminateur à portes 22 et un codeur d'amplitude 24.

La linéarité intégrale et la linéarité différentielle du codeur d'amplitude doivent être suffisantes pour exploiter pleinement les possibilités que permettent l'emploi d'un détecteur de haute résolution.

Le nombre et la disposition des détecteurs dépend de la forme et des dimensions des bagages à contrôler.

Le nombre de détecteurs et donc de chaînes de détection dépend aussi de l'angle solide observable par un détecteur, de la valeur maximum de PF choisie pour caractériser la présence d'un explosif et du nombre de neutrons émis par la source par unité de temps.

Comme on le voit sur la figure 5, les codeurs d'amplitude sont reliés à un calculateur 26 qui est lui-même relié à des moyens de signalisation 28 (aptes à afficher les résultats d'un contrôle et/ou à émettre un signal sonore s'il y a présomption d'explosif dans un bagage contrôlé), le calculateur recevant les valeurs des codages d'amplitude relatives à chaque chaîne.

De préférence, la source 4 est une source pulsée, apte à fournir des bouffées de neutrons ("neutron bursts" dans les publications en langue anglaise) et, comme on le voit sur les figures 4 et 5, cette source pulsée est reliée à chacun des discriminateurs à porte 22 par une ligne de synchronisation 30, en vue d'effectuer la détection des photons gamma prompts en coïncidence avec les bouffées de neutrons rapides et la détection des photons gamma de capture entre ces bouffées de neutrons.

Le système représenté sur les figures 4 et 5 fonctionne de la façon suivante :

un bagage, placé par un tapis roulant 6, pénètre dans l'enceinte de thermalisation 2 et est mis en présence de la source 4 et de la matrice de détecteurs 14 correspondant au tapis roulant. La source émet des neutrons rapides qui sont thermalisés par les parois de l'enceinte de sorte que le bagage est aussi irradié par des neutrons thermiques et émet essentiellement des photons gamma prompts, de capture et d'activation.

Les détecteurs 14 de la matrice reçoivent ces photons gamma et fournissent des signaux aux moyens électroniques de traitement qui les traitent de la façon suivante :

On suppose qu'on a choisi d'examiner des raies de l'azote et des raies de l'oxygène par exemple. Le calculateur a en mémoire la quantité $M'2i$ correspondant à la raie $i$, pour chacune des $n$ raies choisies. Chaque chaîne de détection permet au calculateur 26 d'acquérir une valeur de comptage $Ni$ pour chaque raie $i$. Le calculateur peut ainsi calculer, grâce à la formule (2), la probabilité $PFi$ pour chaque raie $i$ et pour chaque chaîne de détection. Le calculateur 26 calcule alors, pour chaque chaîne de détection, la quantité $PF$ égale au produit des quantités $PFi$ conformément à la formule suivante :

$$PF = \prod_{i=1}^{n} PFi \qquad (10)$$

$PF$ étant la probabilité pour que l'ensemble des comptages effectués sous tous les pics estimés pertinents pour la détection d'explosif soit dû uniquement au bruit ou à une quantité d'explosif inférieure à la quantité maximum présente dans des bagages ne contenant aucun explosif.

Ensuite, pour chaque chaîne de détection, le calculateur 26 compare $PF$ à un seuil $S1$ fixé par les utilisateurs.

Si, pour l'une au moins des chaînes de détection, $PF$ est inférieur à $S1$, le bagage est présumé contenir un explosif et le calculateur commande alors les moyens de signalisation 28. Ce bagage suspect est alors dirigé jusqu'à une zone de stockage des bagages suspects pour y être examiné.

Si, au contraire, $PF$ est au moins égal à $S1$ pour chacune des chaînes de détection, le bagage est considéré comme ne contenant aucun explosif et quitte l'enceinte de thermalisation 2 par la sortie 12 correspondant au tapis roulant sur lequel le bagage se trouve.

Dans une variante de réalisation du système représenté sur les figures 4 et 5, le calculateur calcule, pour chaque chaîne de détection, la probabilité $PFi$ pour chaque raie $i$ puis, par la formule (6), la quantité d'information $Ii$ correspondante. Ensuite, le calculateur calcule la somme de ces quantités d'information (pour chaque chaîne de détection). Chaque somme est ensuite comparée à un seuil $S2$ fixé par les utilisateurs.

Si la somme est supérieure à $S2$ pour l'une quelconque des chaînes de détection, le bagage est présumé contenir un explosif, les utilisateurs sont avertis de ceci et le bagage est dirigé vers la zone des bagages suspects.

Si, au contraire, la somme est inférieure ou égale à $S2$ pour chacune des chaînes de détection, le bagage est considéré comme ne contenant aucun explosif et quitte l'enceinte de thermalisation.

Dans une autre variante de réalisation, au lieu d'utiliser la formule (6) pour calculer la quantité d'information $Ii$, on utilise la formule (7), le calculateur ayant en mémoire les fonctions $K$ et $a$, ce qui conduit à un temps de calcul plus court.

La figure 6 illustre schématiquement d'autres moyens électroniques de traitement des impulsions fournies par les détecteurs 14. Ces autres moyens électroniques de traitement diffèrent de ceux qui sont représentés sur la figure 5 par le fait que la calculateur 26 est remplacé par une pluralité de processeurs 32 qui sont respectivement associés aux chaînes de détection (comportant chacune les éléments 18, 20, 22 et 24), chaque processeur 32 étant placé à la suite du codeur 24 correspondant et relié à ce dernier.

Dans le cas des moyens électroniques de traitement représentés sur la figure 6, chaque processeur 32 calcule le produit $PF$ (ou la somme des quantités d'informations) pour la chaîne de détection à laquelle il est associé.

Tous les processeurs 32 sont reliés à des moyens de signalisation 34 qui permettent d'avertir les utilisateurs du système lorsqu'il s'avère, grâce à l'une au moins des chaînes de détection, qu'il y a présomption de présence d'explosif.

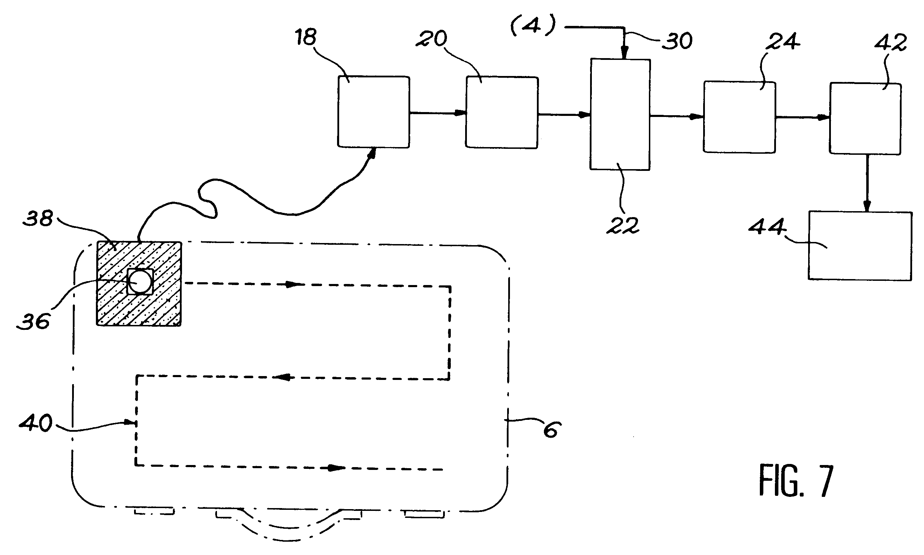

Sur la figure 7, on a représenté schématiquement et partiellement un autre système de détection conforme à l'invention, qui diffère de celui qui est représenté sur la figure 4 par le fait qu'il utilise un seul détecteur de haute résolution 36 au lieu d'un matrice de tels détecteurs, ce détecteur 36 étant placé comme précédemment et logé dans des moyens 38 de collimation et de protection vis-à-vis du rayonnement direct de la source 4.

Des moyens non représentés permettent le déplacement du détecteur 36, associé aux moyens 38, suivant un trajet 40 lui permettant de contrôler la totalité du bagage 6, ce dernier étant immobilisé.

Les signaux fournis par le détecteur 36 sont envoyés à une chaîne de détection composée, comme précédemment, d'un préamplificateur 18, d'un amplificateur proportionnel 20, d'un discriminateur à porte 22 et d'un codeur d'amplitude 24, ce dernier étant relié à un calculateur 42, lui-même relié à des moyens de signalisation 44.

Les moyens de déplacement du détecteur 36 permettent d'immobiliser celui-ci sous le bagage 6 en plusieurs points et, si aucun explosif n'a été détecté en chacun de ces points, le bagage est considéré comme ne contenant pas d'explosif alors que si pour l'un des points, il y a présomption de présence d'explosif, le bagage est dirigé vers la zone des bagages suspects pour y être examiné.

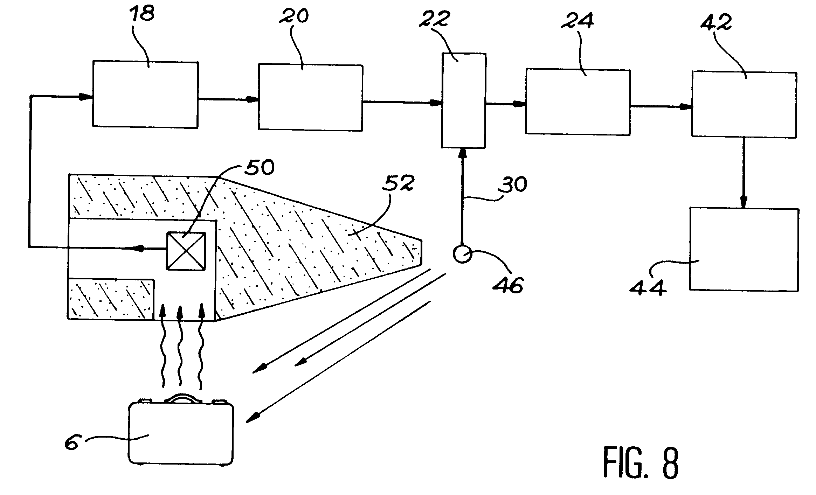

Sur la figure 8, on a représenté schématiquement et partiellement un autre système de détection conforme à l'invention.

Le système représenté sur la figure 8 diffère de celui qui est représenté sur la figure 4 par le fait qu'il a une configuration "ouverte" et donc qu'il ne comporte pas d'enceinte de thermalisation des neutrons.

Le système représenté sur la figure 8 comprend encore une source pulsée 46 de neutrons rapides, mise en présence du bagage à contrôler.

Le système représenté sur la figure 8 comprend également un détecteur 50 de haute résolution pour détecter le rayonnement gamma et ce détecteur est, comme précédemment, relié à une chaîne de détection du genre de celle qui est représentée sur la figure 7, cette chaîne de détection étant reliée à un calculateur, lui-même relié à des moyens de signalisation.

Comme précédemment, la source 46 est reliée par une ligne de synchronisation au discriminateur à porte de la chaîne pour les raisons expliquées plus haut.

De plus, le détecteur 50 est protégé du rayonnement direct de la source 46 et collimaté, par des moyens appropriés 52, vers le bagage 6 à contrôler.

Ainsi, chaque bagage à contrôler se trouve en regard du détecteur 50 collimaté, la source 46 étant placée de façon à pouvoir irradier le bagage 6 par les neutrons rapides qu'elle engendre, de sorte que le bagage 6 émet essentiellement un rayonnement gamma qui est dû aux neutrons rapides et qui arrive au détecteur 50. Les impulsions émises par ce dernier sont traitées par les moyens électroniques de traitement associés à ce détecteur 50.

Bien entendu, au lieu d'un seul détecteur, on pourrait utiliser une matrice de détecteurs, chaque détecteur de la matrice étant associé à une chaîne de détection comme on l'a expliqué en se référant aux figures 5 et 6.

La présente invention n'est pas limitée à la détection d'explosifs nitrés. Elle s'applique également, par exemple, à la détection d'explosifs non nitrés et même à la détection d'un grand nombre de composés chimiques, en exploitant les signatures gamma relatives à des éléments chimiques de ces composés.

L'invention s'applique en particulier à la détection de produits stupéfiants, en exploitant notamment les pics du carbone et de l'azote.

En prenant l'exemple d'un contrôle de bagages pour s'assurer qu'ils ne contiennent pas d'explosif, on explique ci-après l'obtention préalable des paramètres et fonctions (M2i, Ci $1 \leqq i \leqq n$, K et a) qu'il convient de mémoriser pour faire les divers calculs.

Le bruit de fond physique M2i sous le pic i est estimé par extrapolation de la densité de probabilité de part et d'autre de ce pic.

La contribution maximum (Ci) au comptage des matériaux non explosifs du bagage typique est mesurable si l'on dispose d'un nombre suffisant de bagages de voyageurs, remplis de produits habituels (vêtements, objets de toilette ...) : on fait passer de tels bagages (sans explosif) dans le système de la figure 4 (ou on les place successivement devant le système de la figure 8), d'où divers comptages dont on fait la moyenne pour obtenir Ci.

Il est à noter que le calcul de PFi peut être fait directement, à partir de la formule (2), ou bien en tabulant d'une façon ou d'une autre les valeurs de PFi en fonction de Ni et de M2i, par exemple comme cela est expliqué dans le document EP-A-0200596.

Les valeurs de K(M'2i) et de a(M'2i) peuvent être tabulées dans les intervalles utiles de M'2i.

Pour faire une telle tabulation préalable, on détermine des courbes du genre de celles de la figure 1 : en fixant les paramètres M1 et M2, on calcule PF (formule (2)) puis I (formule (6)) et ce, pour diverses valeurs du couple (M1, M2).

A partir des courbes ainsi obtenues et pour diverses valeurs du paramètre M2, on détermine K et a : à une valeur fixée de M2 correspond une courbe du réseau de courbes obtenu ; on choisit deux points P1 et P2 de cette courbe d'où deux couples (I(P1), M1(P1)) et (I(P2 ), M1(P2)) respectivement associés à ces deux points ; à partir de la formule (7) on obtient alors un système de deux équations à deux inconnues dont la solution donne K(M2) et a(M2) pour la valeur fixée de M2.

**Revendications**

1. Système de détection d'une substance susceptible d'être contenue dans un objet (6), système caractérisé en ce qu'il comprend :
   - des moyens (4, 46) d'irradiation de l'objet par des neutrons,
   - des moyens (14, 36, 50) de détection du rayonnement gamma susceptible d'être alors émis par l'objet, et
   - des moyens électroniques (18, 20, 22, 24, 26, 28, 32, 34, 42, 44) de traitement des signaux fournis par les moyens de détection, ces moyens électroniques de traitement étant prévus pour
     . compter les photons gamma correspondant à chaque raie d'un ensemble de raies caractéristiques d'au moins un élément chimique de la substance,
     . déterminer, pour chaque raie i, une probabilité de fausse détection de l'élément chimique associé à cette raie, c'est-à-dire la probabilité PFi pour que le signal détecté, correspondant à cette raie i, soit dû à un bruit de fond,
     . déterminer le produit de ces probabilités de fausse détection,
     . comparer ce produit à un seuil fixé par les utilisateurs du système, et
     . avertir ces utilisateurs si le produit est inférieur au seuil fixé par eux, l'objet étant alors présumé contenir la substance.

2. Système selon la revendication 1, caractérisé en ce que chaque probabilité PFi est déterminée par la formule :

$$PFi = e^{-M'2i} \sum_{j=Ni+1}^{+\infty} \frac{M'2i^{j}}{j\,!}$$

   où Ni représente le comptage, pendant un temps Dt, qui correspond à la raie i et qui est dû à des réactions nucléaires induites par des neutrons et aussi à un bruit de fond M'2i relatif à la raie i, pendant le temps Dt.

3. Système selon la revendication 2, caractérisé en ce que chaque bruit de fond M'2i est déterminé en faisant la somme d'un bruit de fond physique M2i, relatif à la raie i, et de la borne supérieure Ci de comptages relatifs à cette raie i, pendant le temps Dt, sur des objets qui sont susceptibles de contenir la substance mais dont on est sûr qu'ils n'en contiennent pas.

4. Système selon l'une quelconque des revendications 1 à 3, caractérisé en ce que les moyens électroniques de traitement (18, 20, 22, 24, 26, 28, 32, 34, 42, 44) sont prévus pour :
   . déterminer, pour chaque raie i, une quantité d'information Ii relative à cette raie i et définie par la formule :

$$Ii = -\log \frac{PFi}{1-PFi}$$

   . déterminer la somme de ces quantités d'information,
   . comparer cette somme à un seuil fixé par les utilisateurs du système, et
   . avertir ces utilisateurs si cette somme est supérieure au seuil auquel elle est comparée, l'objet étant alors présumé contenir la substance.

**5.** Système selon l'une quelconque des revendications 1 à 3, caractérisé en ce que les moyens électroniques de traitement (18, 20, 22, 24, 26, 28, 32, 34, 42, 44) sont prévus pour :

. déterminer, pour chaque raie i, une quantité d'information Ii relative à cette raie i et définie par la formule :

$$ Ii \ = \ K(M'2i). \frac{N1i^{a(M'2i)}}{M'2i^{\,1/2}} $$

où K et a sont des fonctions mémorisées d'un bruit de fond M'2i relatif à la raie i et N1i est la partie entière de la différence Ni-M'2i, Ni représentant le comptage, pendant un temps Dt, qui correspond à la raie i et qui est dû à des réactions nucléaires induites par des neutrons et aussi au bruit de fond M'2i relatif à la raie i, pendant le temps Dt,

. déterminer la somme de ces quantités d'information,

. comparer cette somme à un seuil fixé par les utilisateurs du système, et

. avertir ces utilisateurs si cette somme est supérieure au seuil auquel elle est comparée, l'objet étant alors présumé contenir la substance.

**6.** Système selon l'une quelconque des revendications 1 à 5, caractérisé en ce que les moyens d'irradiation comprennent :

- une source (4) de neutrons rapides, et
- une enceinte (2) de thermalisation de ces neutrons rapides, dans laquelle se trouve la source et qui est prévue pour recevoir l'objet (6).

**7.** Système selon l'une quelconque des revendications 1 à 5, caractérisé en ce que les moyens d'irradiation comprennent une source (46) de neutrons rapides qui est prévue pour irradier l'objet (6).

**8.** Système selon l'une quelconque des revendications 1 à 7, caractérisé en ce que les moyens de détection comprennent une pluralité de détecteurs (14) de rayonnement gamma et en ce que les moyens électroniques de traitement comprennent une pluralité de chaînes de détection (18, 20, 22, 24), respectivement associées aux détecteurs.

**9.** Système selon l'une quelconque des revendications 1 à 8, caractérisé en ce que les moyens de détection (14, 36, 50) du rayonnement gamma sont des moyens de détection de haute résolution.

**10.** Système selon l'une quelconque des revendications 1 à 9, caractérisé en ce que, les moyens d'irradiation comprenant une source (4, 46) de neutrons, les moyens de détection (14, 36, 50) sont protégés du rayonnement direct de la source et collimatés vers l'objet.

**11.** Système selon l'une quelconque des revendications 1 à 10, caractérisé en ce que les moyens d'irradiation comprennent une source pulsée (4, 46) de neutrons, prévue pour fournir des bouffées de neutrons et en ce que les moyens électroniques de traitement coopérent avec les moyens de détection pour faire des mesures dans des intervalles de temps où l'on est sûr de détecter seulement l'une des catégories de photons gamma engendrés lors de l'irradiation de l'objet par des neutrons.

**12.** Système selon l'une quelconque des revendications 1 à 11, caractérisé en ce que les moyens d'irradiation comprennent une source (4, 46) de neutrons de 14 MeV engendrés par des réactions de fusion.

**Patentansprüche**

**1.** System zum Nachweis einer Substanz, die möglicherweise in einem Objekt (6) enthalten ist, wobei das System dadurch gekennzeichnet ist, daß es

- Einrichtungen (4, 46) zur Bestrahlung des Objekts mit Neutronen,
- Einrichtungen (14, 36, 50) zur Detektion der Gamma-Strahlung , die dann möglicherweise durch das Objekt emittiert wird, und
- elektronische Einrichtungen (18, 20, 22, 24, 26, 28, 32, 34, 42, 44) zur Verarbeitung der von den Detektionseinrichtungen gelieferten Signale umfaßt, wobei die elektronischen Einrichtungen dazu vorgesehen sind,

. die jedem Peak einer Gruppe von charakteristischen Peaks wenigstens eines chemischen Elements der Substanz entsprechenden Gamma-Photonen zu zählen,

. für jeden Peak i eine Wahrscheinlichkeit für den fälschlichen Nachweis des diesem Peak zugeordneten chemischen Elements zu bestimmen, d.h. die Wahrscheinlichkeit PFi dafür, daß das detektierte Signal, das diesem Peak entspricht, vom Untergrundrauschen herrührt,

. das Produkt dieser Wahrscheinlichkeiten für den fälschlichen Nachweis zu bestimmen,

. dieses Produkt mit einer von den Benutzern des Systems festgelegten Schwelle zu vergleichen, und

. diesen Benutzern zu melden, wenn das Produkt kleiner als die von ihnen festgelegte Schwelle ist, wobei dann anzunehmen ist, daß das Objekt die Substanz enthält.

2. System nach Anspruch 1, dadurch gekennzeichnet, daß jede Wahrscheinlichkeit PFi bestimmt wird durch die Formel

$$PFi = e^{-M'2i} \sum_{j=Ni+1}^{+\infty} \frac{M'2i^j}{j!} \,,$$

wo Ni das dem Peak i entsprechende Zählresultat während einer Zeit Dt bedeutet, verursacht durch neutroneninduzierte Kernreaktionen und auch durch Untergrundrauschen M'2i bezüglich des Peaks i während der Zeit Dt.

3. System nach Anspruch 2, dadurch gekennzeichnet, daß jedes Untergrundrauschen M'2i dadurch bestimmt wird, daß man die Summe eines physikalischen Untergrundrauschens M2i bezüglich des Peaks i und der oberen Grenze Ci der Zählresultate bezüglich dieses Peaks i während der Zeit Dt bildet, für Objekte, die grundsätzlich die Substanz enthalten können, von denen aber feststeht, daß sie sie nicht enthalten.

4. System nach einem der Ansprüche 1 bis 3, dadurch gekennzeichnet, daß die elektronischen Einrichtungen zur Verarbeitung (18, 20, 22, 24, 26, 28, 32, 34, 42, 44) dazu vorgesehen sind,

. für jeden Peak i eine Informationsgröße Ii bezüglich dieses Peaks zu bestimmen, die definiert ist durch die Formel

$$Ii = -\log \frac{PFi}{1 - PFi} \,,$$

. die Summe dieser Informationsgrößen zu bestimmen,

. diese Summe mit einer von den Benutzern des Systems festgelegten Schwelle zu vergleichen, und

. den Benutzern zu melden, wenn diese Summe größer ist als die Schwelle, mit der sie verglichen wird, wobei dann anzunehmen ist, daß das Objekt die Substanz enthält.

5. System nach einem der Ansprüche 1 bis 3, dadurch gekennzeichnet, daß darin die elektronischen Mittel zur Verarbeitung (18, 20, 22, 24, 26, 28, 32, 34, 42, 44) dazu vorgesehen sind,

. für jeden Peak i eine Informationsgröße Ii bezüglich dieses Peaks zu bestimmen, die definiert ist durch die Formel

$$Ii = K(M'2i) \cdot \frac{N1i^{a(M'2i)}}{M'2i^{1/2}} \,,$$

wo K und a gespeicherte Funktionen eines Untergrundrauschens M'2i bezüglich des Peaks i sind und N1i der ganzzahlige Teil der Differenz Ni - M'2i ist, wobei Ni das dem Peak i entsprechende Zählresultat während einer Zeit Dt bedeutet, verursacht durch neutroneninduzierte Kernreaktionen und auch durch Untergrundrauschen M'2i bezüglich des Peaks i während der Zeit Dt,

. die Summe dieser Informationsgrößen zu bestimmen,

. diese Summe mit einer von den Benutzern des Systems festgelegten Schwelle zu vergleichen, und

. den Benutzern zu melden, wenn diese Summe größer ist als die Schwelle, mit der sie verglichen wird, so daß dann anzunehmen ist, daß das Objekt die Substanz enthält.

6. System nach einem der Ansprüche 1 bis 5, dadurch gekennzeichnet, daß darin die Bestrahlungseinrichtungen

- eine Quelle (4) schneller Neutronen und

- einen Behälter (2) zur Thermalisierung dieser schnellen Neutronen umfassen, in dem sich die Quelle befindet und der dazu vorgesehen ist, das Objekt (6) aufzunehmen.

**7.** System nach einem der Ansprüche 1 bis 5, dadurch gekennzeichnet, daß die Bestrahlungseinrichtungen eine Quelle (46) schneller Neutronen umfassen, die dazu vorgesehen ist, das Objekt (6) zu bestrahlen.

**8.** System nach einem der Ansprüche 1 bis 7, dadurch gekennzeichnet, daß die Detektionseinrichtungen eine Mehrzahl von Detektoren (14) für Gammastrahlung umfassen und daß die elektronischen Einrichtungen zur Verarbeitung eine Mehrzahl von Detektionskanälen (18, 20, 22, 24) umfassen, die jeweils den Detektoren zugeordnet sind.

**9.** System nach einem der Ansprüche 1 bis 8, dadurch gekennzeichnet, daß die Detektionseinrichtungen (14, 36, 50) für die Gammastrahlung Detektionseinrichtungen hoher Auflösung sind.

**10.** System nach einem der Ansprüche 1 bis 9, dadurch gekennzeichnet, daß, da die Bestrahlungseinrichtungen eine Neutronenquelle (4, 46) umfassen, die Detektionseinrichtungen (14, 36, 50) vor der direkten Strahlung der Quelle geschützt und auf das Objekt kollimiert sind.

**11.** System nach einem der Ansprüche 1 bis 10, dadurch gekennzeichnet, daß die Bestrahlungseinrichtungen eine gepulste Neutronenquelle umfassen, dazu vorgesehen, Pakete von Neutronen zu liefern, und dadurch, daß die elektronischen Einrichtungen zur Verarbeitung mit den Detektionseinrichtungen zusammenwirken, um Messungen in den Zeitintervallen zu machen, in denen man sicher ist, ausschließlich eine der bei der Bestrahlung des Objekts mit Neutronen erzeugten Kategorien von Gamma-Photonen zu detektieren.

**12.** System nach einem der Ansprüche 1 bis 11, dadurch gekennzeichnet, daß die Bestrahlungseinrichtungen eine Quelle (4, 46) von Neutronen von 14 MeV, erzeugt durch Fusionsreaktionen, umfassen.

## Claims

**1.** System for detecting a substance liable to be contained in an object (6), characterized in that it comprises: means (4,46) for irradiating the object by neutrons,
means (14,36,50) for detecting the gamma radiation which can then be emitted by the object and electronic means (18,20,22,24,26,28,32,34,42,44) for the processing of the signals supplied by the detection means, said electronic processing means being provided for
counting the gamma photons corresponding to each line of a plurality of characteristic lines of at least one chemical element of the substance,
determining, for each line i, a false detection probability for the chemical element associated with said line, i.e. the probability PFi that the detected signal, corresponding to the said line i, is due to a background noise,
determining the product of these false detection probabilities,
comparing said product with a threshold fixed by the system users and
notifying said users if the product is below the threshold fixed by them, the object then being assumed to contain the substance.

**2.** System according to claim 1, characterized in that each probability PFi is determined by the formula:

$$PFi = e^{-M'2i} \sum_{j=Ni+1}^{+\infty} \frac{M'2i^{j}}{j!}$$

in which Ni represents the count during a time Dt, which corresponds to the line i and which is due to the nuclear reactions induced by the neutrons and also a background noise M'2i relative to the line i and for the time Dt.

**3.** System according to claim 2, characterized in that each background noise M'2i is determined by forming the sum of a physical background noise M2i, relative to the line i, and the upper limit Ci of the counts relative to the said line i, during the time Dt, on objects liable to contain the substance, but known not to contain the substance.

4. System according to any one of the claims 1 to 3, characterized in that the electronic processing means (18,20,22,24,26,28,32, 34,42,44) are provided for:
determining, for each line i, an information quantity Ii relative to line i and defined by the formula:

$$Ii = - \log \frac{PFi}{1 - PFi}$$

determining the sum of these information quantities,
comparing said sum with a threshold fixed by the system users and
notifying said users if said sum exceeds the threshold with which it is compared, the object then being assumed to contain the substance.

5. System according to any one of the claims 1 to 3, characterized in that the electronic processing means (18,20,22,24,26,28,32, 34,42,44) are provided for:
determining, for each line i, an information quantity Ii relative to said line i and defined by the formula:

$$Ii = K(M'2i). \frac{N1i^{a(M'2i)}}{M'2i^{1/2}}$$

in which K and a are stored functions of a background noise M'2i relative to the line i and N1i is the integral part of the difference Ni-M'2i, Ni representing the count during a time Dt corresponding to the line i and which is due to the nuclear reactions induced by the neutrons and also to the background noise M'2i relative to the line i and for the time Dt,
determining the sum of these information quantities,
comparing said sum with a threshold fixed by the system users and
notifying said users if said sum exceeds the threshold with which it is compared, the object then being assumed to contain the substance.

6. System according to any one of the claims 1 to 5, characterized in that the irradiation means comprise a fast neutron source (4) and an enclosure (2) for thermalizing these fast neutrons, in which is located the source and which serves to receive the object (6).

7. System according to any one of the claims 1 to 5, characterized in that the irradiation means incorporate a fast neutron source (46) for irradiating the object (6).

8. System according to any one of the claims 1 to 7, characterized in that the detection means comprise a plurality of gamma radiation detectors (14) and that the electronic processing means comprise a plurality of detection chains (18,20,22,24) respectively associated with the detectors.

9. System according to any one of the claims 1 to 8, characterized in that the gamma radiation detection means (14,36,50) are high resolution detection means.

10. System according to any one of the claims 1 to 9, characterized in that the irradiation means incorporate a neutron source (4,46), the detection means (14,36,50) being detected from the direct radiation from the source and are collimated towards the object.

11. System according to any one of the claims 1 to 10, characterized in that the irradiation means comprise a pulsed neutron source (4,46) for supplying neutron bursts and in that the electronic processing means cooperate with the detection means in order to carry out measurements in the time intervals when it is certain that detection will only take place of one of the categories of gamma photons produced during the irradiation of the object by neutrons.

12. System according to any one of the claims 1 to 11, characterized in that the irradiation means comprise a source (4,46) of 14 MeV neutrons produced by fusion reactions.

14

FIG. 1

FIG. 2

FIG. 3

FIG. 4

EP 0 446 333 B1

FIG. 5

FIG. 6

FIG. 7

EP 0 446 333 B1

FIG. 8